# EUROPÄISCHE PATENTSCHRIFT

(11) **EP 3 250 294 B1**
(45) Veröffentlichungstag und Bekanntmachung des Hinweises auf die Patenterteilung: **15.05.2019**
(21) Anmeldenummer: 15793842.4
(22) Anmeldetag: 12.11.2015
(51) Int. Cl.: A61Q 15/00, A61K 8/365, A61K 8/44

(54) **ANTITRANSPIRANTIEN, WELCHE SALIZYLSÄURE UND AMINOPOLYCARBOXYLAT DERIVATE ENTHALTEN**
ANTIPERSPIRANTS CONTAINING SALICYLIC ACID AND AMINOPOLYCARBOXYLIC ACID DERIVATIVES
ANTITRANSPIRANT CONTENANT ACIDE SALICYLIQUE ET DES DERIVES ACIDES AMINOPOLYCARBOXYLIQUES

(30) Priorität: 30.01.2015 DE 102015201653
(43) Veröffentlichungstag der Anmeldung: 06.12.2017
(73) Patentinhaber: Henkel AG & Co. KGaA, 40589 Düsseldorf (DE)
(72) Erfinder: DÖRING, Thomas, 41540 Dormagen (DE)
(86) Internationale Anmeldenummer: PCT/EP2015/076448
(87) Internationale Veröffentlichungsnummer: WO 2016/119937

(56) Entgegenhaltungen:
- WO-A1-2006/056283
- WO-A2-2013/174725

## Beschreibung

Die vorliegende Erfindung betrifft ein schweißhemmendes kosmetisches Mittel, welches mindestens eine schweißhemmende Verbindung, gegebenenfalls mindestens ein Treibmittel sowie spezielle weitere Verbindungen enthält. Durch den Zusatz dieser Verbindungen kann die Menge der schweißhemmenden Verbindung reduziert werden, ohne die schweißhemmende Wirkung des kosmetischen Mittels negativ zu beeinflussen.

Weiterhin betrifft die vorliegende Erfindung eine Verpackungseinheit (Kit-of-parts) enthaltend ein erfindungsgemäßes kosmetisches Mittel sowie ein kosmetisches Mittel mit mindestens einer schweißhemmenden Verbindung.

Zudem betrifft die vorliegende Erfindung die Verwendung einer Kombination, welche mindestens eine schweißhemmende Verbindung, gegebenenfalls mindestens ein Treibmittel sowie spezielle weitere Verbindungen enthält, zur Reduzierung und/oder Verhinderung von Schweiß, insbesondere Achselschweiß oder Schweiß anderer Körperregionen.

Schließlich betrifft die vorliegende Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder der Inhalt einer erfindungsgemäßen Verpackungseinheit auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher). Kosmetische Antitranspirantien des Standes der Technik enthalten neben mindestens einem Öl oder einem Wachs und einer Riechstoffkomponente bzw. einem Parfüm mindestens eine schweißhemmende Verbindung, insbesondere in Form von Halogeniden und/oder Hydroxyhalogeniden von Aluminium und/oder Zirconium. Diese schweißhemmenden Verbindungen verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Halogenide und/oder Hydroxyhalogenide von Aluminium und/oder Zirconium können in Verbindung mit dem sauren pH-Wert dieser Antitranspirantien bei einigen Anwendern zu unangenehmen Hautreaktionen führen. Darüber hinaus kann die Verwendung der vorgenannten schweißhemmenden Verbindungen zu einer Fleckenbildung auf der Kleidung führen, siehe hierzu z.B. WO2006056283 A1.

Es besteht daher ein Bedarf die Gesamtmenge an schweißhemmenden Halogeniden und/oder Hydroxyhalogeniden von Aluminium und/oder Zirconium in schweißhemmenden kosmetischen Mitteln zu verringern. Diese schweißhemmenden kosmetischen Mittel sollen eine gute schweißhemmende Wirkung, eine gute Hautverträglichkeit sowie eine hohe Lagerstabilität aufweisen.

Der vorliegenden Erfindung lag die Aufgabe zugrunde, ein schweißhemmendes kosmetisches Mittel bereitzustellen, welches die Nachteile des Standes der Technik vermeidet bzw. zumindest abschwächt und welches eine gute Hautverträglichkeit bei gleichzeitig zuverlässiger Verminderung der Achselnässe besitzt. Darüber hinaus soll das schweißhemmende kosmetische Mittel eine hohe Lagerstabilität aufweisen.

Es wurde nun überraschend gefunden, dass durch den Einsatz von Salicylsäure und speziellen stickstoffhaltigen Verbindungen in kosmetischen Mitteln die Gesamtmenge an schweißhemmenden Verbindungen deutlich verringert werden kann, ohne dass die schweißhemmende Wirkung dieser Mittel oder deren Lagerstabilität negativ beeinflusst wird.

Gegenstand der vorliegenden Erfindung ist somit ein schweißhemmendes kosmetisches Mittel, enthaltend -jeweils bezogen auf das Gesamtgewicht des schweißhemmenden kosmetischen Mittels
a) 1 bis 30 Gew.-% mindestens eine schweißhemmende Verbindung,
b) 0,1 bis 10 Gew.-% Salicylsäure
c) mindestens eine Verbindung der Formel (I) 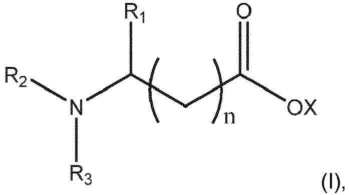
in der
- n: für 0 oder 1 steht,
- X: für -H, Na⁺, K⁺ oder NH4⁺ steht,
- R₁: ausgewählt ist aus-H, -COOX oder -(CH₂)-COOX steht,
- R₂: ausgewählt ist aus -H, C₁₋₁₀-Alkyl oder COOX,
- R₃: ausgewählt ist aus -(CH₂)-COOX, -CH(R₁)-COOX, -(CH₂)₂-NH[CH(COOX)(CH₂COOX)], -CH(R₄)-C(R₅)(R₆)-COOX

- R₄: ausgewählt ist aus -H, C₁₋₁₀-Alkyl oder COOX,
- R₅, R₆: unabhängig voneinander -H, -OH oder C₁₋₁₀-Alkyl sind.

Die erfindungsgemäßen Mittel enthalten 1 bis 30 Gew.-% mindestens eine schweißhemmende Verbindung. Mit besonderem Vorzug enthalten schweißhemmende Mittel Aluminium Chlorohydrat oder Aluminium Zirkonium Chlorohydrat. Diese Verbindungen entstehen in wässriger Lösung durch Hydrolyse der Al(all)- und Zr(IV) Aqua-Komplexe im pH-Bereich von 4 bis 7. In beiden Fällen entstehen oligomere und polymere Komplexe mit Hydroxid- und Oxid-Brücken zwischen den Metallionen. In hohen Konzentrationen ist die Tendenz zur Bildung hochmolekularer Komplexe (2000-10000 Da) sehr stark ausgeprägt. Die schweißhemmende Wirksamkeit ist hier nur sehr schwach. Kleinere Komplexkationen (500-1500 Da), das Monomer AlCl₃ x 6 H20 und das Dimer Al₂(OH)₂(H20)⁴⁺ sind deutlich besser wirksam. In verdünnten wässrigen Lösungen kann man das Gleichgewicht in Richtung der kleinen Polymere verlagern, allerdings reicht dann die Gesamtkonzentration nicht mehr aus, um einen ausreichenden schweißhemmenden Effekt zu erzielen.

Werden wässrige Lösungen von Aluminiumchlorid nicht neutralisiert, so findet hier praktisch keine Hydrolyse statt und Al(H₂O)₆Cl₃ verbleibt weitgehend in der monomeren Konfiguration. Solche Lösungen zeigen ausgeprägte schweißhemmende Wirksamkeit und werden u.a. für verschreibungspflichtige Antitranspirantien bei Hyperhidrose eingesetzt. Die Hautverträglichkeit dieser Lösungen ist allerdings sehr schlecht und für kosmetische Produkte nicht ausreichend.

Konzentrierte Lösungen von Aluminium Chlorohydrat oder Aluminium Zirkonium Chlorohydrat können durch Zusatz von Salicylsäure und Verbindung(en) der Formel (I) in ihrer schweißhemmenden Wirksamkeit deutlich gesteigert werden. Ohne durch die Theorie beschränkt sein zu wollen, vermutet die Anmelderin, dass die Kombination aus Salicylsäure und Verbindung(en) der Formel (I) in der Lage ist, kleine Komplexkationen zu stabilisieren, so dass die Zusammenlagerung zu hochmolekularen Komplexen eingeschränkt wird. Auf diese Weise entsteht eine hochwirksame wässrige Formulierung mit ausgezeichneter Hautverträglichkeit, ohne dass Aluminiumchlorid zugesetzt werden müsste.

Unter dem Begriff "schweißhemmend" wird erfindungsgemäß die Verminderung bzw. Reduzierung der Transpiration der Schweißdrüsen des Körpers verstanden.
Darüber hinaus werden unter dem Begriff "schweißhemmende Verbindung" im Rahmen der vorliegenden Erfindung Halogenide und/oder Hydroxyhalogenide von Aluminium und/oder Zirconium, insbesondere Chloride, Bromide und lodide des Aluminiums und des Zirconiums, sowie Verbindungen der Formeln Al(OH)_{y}X und Zr(OH)_{z}X verstanden, wobei X in den vorgenannten Formeln für ein Halogenidion steht. Weiterhin werden hierunter Komplexe aus Halogeniden und/oder Hydroxyhalogeniden des Aluminiums und/oder Zirconiums mit Liganden, wie beispielsweise Aminosäuren, Betainen und Hydroxyalkansäuren, verstanden.

Als Bestandteil a) enthalten die erfindungsgemäßen schweißhemmenden kosmetischen Mittel mindestens eine schweißhemmende Verbindung in einer Gesamtmenge von 1 bis 30 Gew.-%, bezogen auf das Gesamtgewicht des kosmetischen Mittels.

Im Rahmen der vorliegenden Erfindung kann die schweißhemmende Verbindung ausgewählt sein aus der Gruppe von (i) wasserlöslichen adstringierenden anorganischen Salzen des Aluminiums, insbesondere Aluminiumchlorhydrat, Aluminiumsesquichlorohydrat, Aluminiumdichlorohydrat, Aluminium-hydroxid, Kaliumaluminiumsulfat, Aluminiumbromhydrat, Aluminiumchlorid, Aluminiumsulfat; (ii) wasserlöslichen adstringierenden organischen Salzen des Aluminiums, insbesondere Aluminiumchlorhydrex-Propylenglycol, Aluminiumchlorhydrex-Polyethylenglycol, Aluminium-Propylenglycol-Komplexe, Aluminiumsesquichlorhydrex-Propylenglycol, Aluminiumsesquichlorhydrex-Polyethylenglycol, Aluminium-Propylenglycol-dichlorhydrex, Aluminium-Polyethylenglycol-dichlorhydrex, Aluminiumundecylenoylcollagen-aminosäure, Natriumaluminium-lactat, Natriumaluminiumchlorhydroxylactat, Aluminium-lipoaminosäuren, Aluminiumlactat, Aluminiumchlorohydroxyallantoinat, Natrium-Aluminium-Chlorohydroxylactat; (iii) wasserlöslichen adstringierenden anorganischen Aluminium-Zirkonium-Salzen, insbesondere Aluminiumzirconiumtrichlorhydrat, Aluminiumzirconiumtetrachlorhydrat, Aluminiumzirconiumpentachlorhydrat, Aluminiumzirconiumoctachlorhydrat; (iv) wasserlöslichen adstringierenden organischen Aluminium-Zirkonium-Salzen, insbesondere Aluminiumzirkonium-Propylenglycol-Komplexe, Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin; sowie (v) deren Mischungen.

Besonders bevorzugte erfindungsgemäße schweißhemmende kosmetische Mittel sind dadurch gekennzeichnet, daß sie 2 bis 25 Gew.-%, vorzugsweise 3 bis 24 Gew.-%, bevorzugt 4 bis 23 Gew.-%, weiter bevorzugt 5 bis 20 Gew.-%, noch mehr bevorzugt 6 bis 19 Gew.-% und insbesondere 8 bis 18 Gew.-% Aluminium-Chlorohydrat enthalten.

Zusätzlich zu Aluminium-Chlorohydrat oder an seiner Stelle können weiter bevorzugte Mittel auch Aluminium-Zirkonium-Salze enthalten. Entsprechend besonders bevorzugte schweißhemmende kosmetische Mittel sind dadurch gekennzeichnet, dass sie 2,5 bis 29 Gew.-%, vorzugsweise 5 bis 28 Gew.-%, bevorzugt 7,5 bis 27 Gew.-%, weiter bevorzugt 10 bis 26 Gew.-%, noch mehr bevorzugt 12,5 bis 25 Gew.-% und insbesondere 15 bis 23 Gew.-% Aluminium-Zirkonium-Chlorohydrat enthalten.

Unter dem Begriff "schweißhemmende Aluminiumsalze" werden erfindungsgemäß keine Alumosilicate und Zeolithe verstanden. Weiterhin werden erfindungsgemäß unter wasserlöslichen Aluminiumsalzen solche Salze verstanden, welche eine Löslichkeit von mindestens 3 Gew.-% bei 20 °C aufweisen, d. h. es lösen sich mindestens 3 g des schweißhemmenden Aluminiumsalzes in 97 g Wasser bei 20 °C.

Eine besonders gute schweißhemmende Wirkung wird im Rahmen der vorliegenden Erfindung erhalten, wenn die schweißhemmende(n) Verbindung(en) in einer Gesamtmenge von 2 bis 20 Gew.-%, vorzugsweise von 2,5 bis 18 Gew.-%, bevorzugt von 3 bis 15 Gew.-%, weiter bevorzugt von 3,5 bis 12 Gew.-%, noch mehr bevorzugt von 4 bis 10 Gew.-%, insbesondere von 4,5 bis 8 Gew.-%, bezogen auf das Gesamtgewicht des schweißhemmenden kosmetischen Mittels, enthalten ist.

Gemäß einer Ausführungsform der vorliegenden Erfindung kann es vorgesehen sein, dass in den erfindungsgemäßen schweißhemmenden kosmetischen Mittel ein Treibmittel eingesetzt wird. Wenn die erfindungsgemäßen kosmetischen Mittel ein Treibmittel enthalten, ist dieses bevorzugt in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%, bezogen auf das Gesamtgewicht des schweißhemmenden kosmetischen Mittels, enthalten. In diesem Fall sind die erfindungsgemäßen kosmetischen Mittel als treibgasgetriebene Aerosole konfektioniert. Bevorzugte Treibmittel (Treibgase) sind Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuß vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Als zweiten wesentlichen Inhaltsstoff enthalten die erfindungsgemäßen Mittel Salicylsäure in Mengen von 0,1 bis 10 Gew.-%, jeweils bezogen auf das anwendungsbereite Mittel - ohne ggf. vorhandene Treibmittel. Besonders bevorzugt ist es, Salicylsäure innerhalb engerer Mengenbereiche einzusetzen, so dass bevorzugte erfindungsgemäße schweißhemmende kosmetische Mittel 0,11 bis 8 Gew.-%, vorzugsweise 0,12 bis 6 Gew.-%, bevorzugt 0,13 bis 4 Gew.-%, weiter bevorzugt 0,14 bis 3 Gew.-%, noch mehr bevorzugt 0,15 bis 2 Gew.-% und insbesondere 0,2 bis 1 Gew.-% Salicylsäure enthalten.

Als dritten wesentlichen Inhaltsstoff enthalten die erfindungsgemäßen Mittel mindestens eine Verbindung der Formel (I) 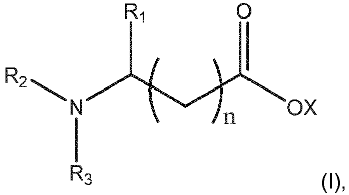 in der
- n: für 0 oder 1 steht,
- X: für -H, Na⁺, K⁺ oder NH4⁺ steht,
- R₁: ausgewählt ist aus-H, -COOX oder -(CH₂)-COOX steht,
- R₂: ausgewählt ist aus -H, C₁₋₁₀-Alkyl oder COOX,
- R₃: ausgewählt ist aus -(CH₂)-COOX, -CH(R₁)-COOX, -(CH₂)₂-NH[CH(COOX)(CH₂COOX)], -CH(R₄)-C(R₅)(R₆)-COOX
- R₄: ausgewählt ist aus -H, C₁₋₁₀-Alkyl oder COOX,
- R₅, R₆: unabhängig voneinander -H, -OH oder C₁₋₁₀-Alkyl sind.

Besonders bevorzugte Verbindungen der Formel (I) sind solche, in denen n für 0 und R₁ für -H steht, womit sich Formel (I) zu Formel (I-1) vereinfacht: 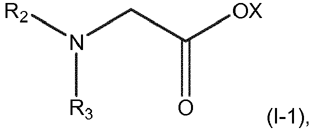 in der
- X: für -H, Na⁺, K⁺ oder NH4⁺ steht,
- R₂: ausgewählt ist aus -H, C₁₋₁₀-Alkyl oder COOX,
- R₃: ausgewählt ist aus -(CH₂)-COOX, -CH(R₁)-COOX, -(CH₂)₂-NH[CH(COOX)(CH₂COOX)], -CH(R₄)-C(R₅)(R₆)-COOX
- R₄: ausgewählt ist aus -H, C₁₋₁₀-Alkyl oder COOX,
- R₅, R₆: unabhängig voneinander -H, -OH oder C₁₋₁₀-Alkyl sind.

In einer weiter bevorzugten Ausführungsform steht R₂ in Formel (I) für -(CH₂)-COOX womit sich Formel (I) zu Formel (I-2) vereinfacht: 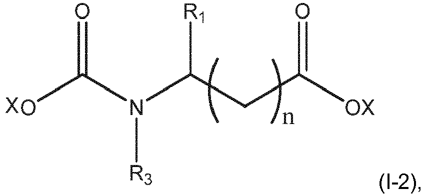 in der
- n: für 0 oder 1 steht,
- X: für -H, Na⁺, K⁺ oder NH4⁺ steht,
- R₁: ausgewählt ist aus-H, -COOX oder -(CH₂)-COOX steht,
- R₃: ausgewählt ist aus -(CH₂)-COOX, -CH(R₁)-COOX, -(CH₂)₂-NH[CH(COOX)(CH₂COOX)], -CH(R₄)-C(R₅)(R₆)-COOX
- R₄: ausgewählt ist aus -H, C₁₋₁₀-Alkyl oder COOX,
- R₅, R₆: unabhängig voneinander -H, -OH oder C₁₋₁₀-Alkyl sind.

Diese bevorzugte Ausführungsform ist auch für Formel (I-1) bevorzugt, womit sich Formel (I-1) zu Formel (I-1a) vereinfacht: 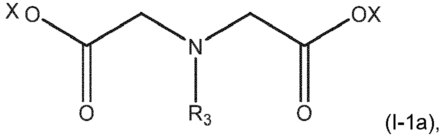 in der
- X: für -H, Na⁺, K⁺ oder NH4⁺ steht,
- R₃: ausgewählt ist aus -(CH₂)-COOX, -CH(R₁)-COOX, -(CH₂)₂-NH[CH(COOX)(CH₂COOX)], -CH(R₄)-C(R₅)(R₆)-COOX
- R₄: ausgewählt ist aus -H, C₁₋₁₀-Alkyl oder COOX,
- R₅, R₆: unabhängig voneinander -H, -OH oder C₁₋₁₀-Alkyl sind.

In einer weiter bevorzugten Ausführungsform steht R₃ in Formel (I) für -(CH₂)₂-N[(CH₂)-COOX]₂ womit sich Formel (I) zu Formel (I-3) vereinfacht: 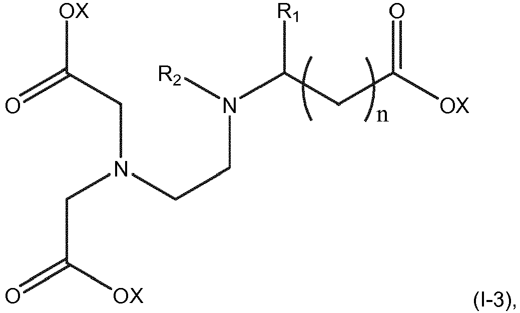 in der
- n: für 0 oder 1 steht,
- X: für -H, Na⁺, K⁺ oder NH4⁺ steht,
- R₁: ausgewählt ist aus-H, -COOX oder -(CH₂)-COOX steht,
- R₂: ausgewählt ist aus -H, C₁₋₁₀-Alkyl oder COOX,
- R₄: ausgewählt ist aus -H, C₁₋₁₀-Alkyl oder COOX,
- R₅, R₆: unabhängig voneinander -H, -OH oder C₁₋₁₀-Alkyl sind.

Diese bevorzugte Ausführungsform ist auch für die Formeln (I-1) und (I-2) bevorzugt, womit sich Formel (I-1) zu Formel (I-1b) und Formel (I-2) zu Formel (I-2a) vereinfacht: 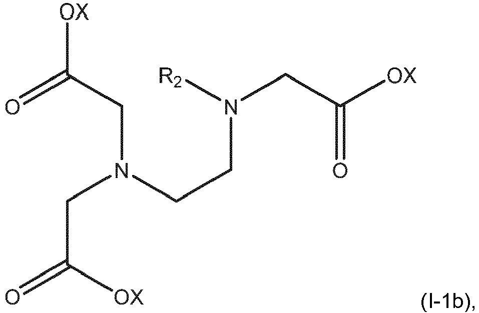 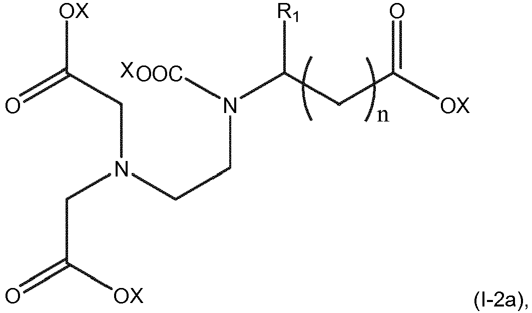 in denen
- n: für 0 oder 1 steht,
- X: für -H, Na⁺, K⁺ oder NH4⁺ steht,
- R₁: ausgewählt ist aus-H, -COOX oder -(CH₂)-COOX steht,
- R₂: ausgewählt ist aus -H, C₁₋₁₀-Alkyl oder COOX.

In einer ebenfalls bevorzugte Ausführungsform steht in Formel (I) n für 0, R₁ für -H, R₂ für -(CH₂)-COOX und R₃ für-(CH₂)-COOX. Erfindungsgemäß bevorzugte schweißhemmende kosmetische Mittel sind daher dadurch gekennzeichnet, dass sie 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (Ib) enthalten 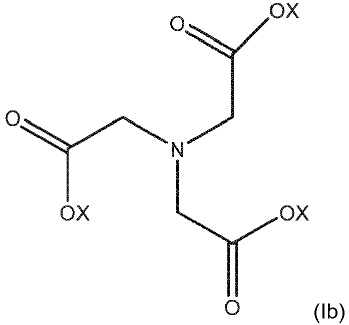 in der X für -H, Na⁺, K⁺ oder NH4⁺ steht.

In einer ebenfalls bevorzugte Ausführungsform steht in Formel (I) n für 0, R₁ für -(CH₂)-COOX und R₃ für -CH(R₁)-COOX. Erfindungsgemäß bevorzugte schweißhemmende kosmetische Mittel sind daher dadurch gekennzeichnet, dass sie 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (Ic) enthalten 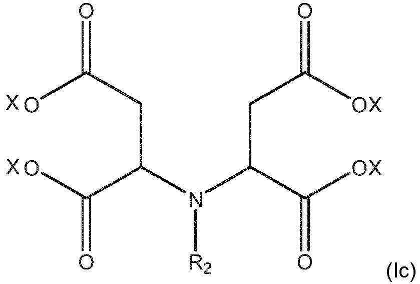 in der X für -H, Na⁺, K⁺ oder NH4⁺ steht und R₂ ausgewählt ist aus -H oder -(CH₂)-COOX.

Eine weitere Gruppe bevorzugter Verbindungen der Formel (I) ist dadurch gekennzeichnet, dass R₃ für -(CH₂)₂-NH[CH(COOX)(CH₂COOX)] steht. Erfindungsgemäß bevorzugte schweißhemmende kosmetische Mittel sind daher dadurch gekennzeichnet, dass sie 0,012 bis 10 Gen.-%, vorzugsweise 0,05 bis 5 Gen.-%, bevorzugt 0,1 bis 2,5 Gen.-%, weiter bevorzugt 0,15 bis 1 Gen.-%, noch mehr bevorzugt 0,2 bis 0,75 Gen.-% und insbesondere 0,25 bis 0,5 Gen.-% Verbindung(en) der Formel (Id) enthalten 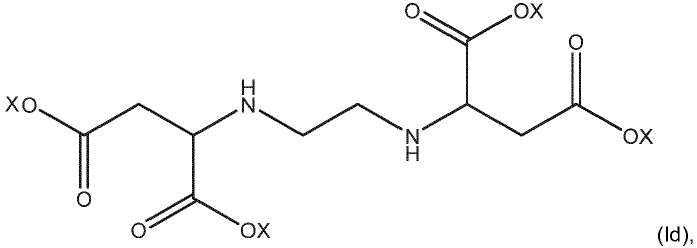 in der X für -H, Na⁺, K⁺ oder NH4⁺ steht.

In allen vorstehen genannten Formeln kann X für ein Wasserstoffatom (freie Säure) oder für Na⁺, K⁺ oder NH4⁺ stehen. Auch teilneutralisierte Formen, in denen jedes X unabhängig voneinander aus den vorstehend genannten Möglichkeiten ausgewählt ist und mindestens ein X für -H steht, sind erfindungsgemäß einsetzbar. Besonders bevorzugt sind die Alkalimetllsalze und darunter die Natriumsalze, d.h. diejenigen Vertreter, in denen jedes X für Na⁺ steht.

Eine weitere Gruppe bevorzugter Verbindungen der Formel (I) ist dadurch gekennzeichnet, dass R₃ für -CH(R₄)-C(R₅)(R₆)-COOX steht. Erfindungsgemäß bevorzugte schweißhemmende kosmetische Mittel sind daher dadurch gekennzeichnet, dass sie 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (le) enthalten 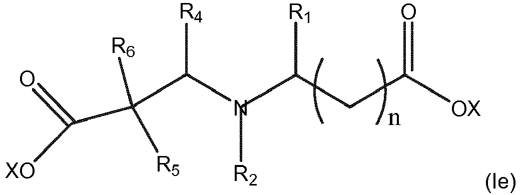 in der
- n: für 0 oder 1 steht,
- X: für -H, Na⁺, K⁺ oder NH4⁺ steht,
- R₁: für -H oder -(CH₂)-COOX steht
- R₂: ausgewählt ist aus -H oder -(CH₂)-COOX,
- R₄: ausgewählt ist aus -H, C₁₋₁₀-Alkyl oder COOX,
- R₅, R₆: unabhängig voneinander -H, -OH oder C₁₋₁₀-Alkyl sind.

"Alkyl", wie hierin verwendet, bezieht sich auf gesättigte geradkettige oder verzweigte Kohlenwasserstoffe mit 1-10, vorzugsweise 1-4 Kohlenstoffatomen und schließt ein, ist aber nicht beschränkt auf Methyl, Ethyl, n-Propyl, iso-Propyl, n-Butyl, sec-Butyl, iso-Butyl und tert-Butyl.

Insbesondere bevorzugte schweißhemmende kosmetische Mittel dieser Ausführungsform sind dadurch gekennzeichnet, dass die Verbindung(en) der Formel (Id) ausgewählt ist/sind aus der Gruppe N-(1,2-Dicarboxy-2-hydroxyethyl)-glycin, N-(1,2-Dicarboxy-2-hydroxyethyl)-alanin, N,N-Biscarboxymethyl-β-alanin, N-(1,2-Dicarboxy-2-hydroxyethyl)-sarkosin, N-(1,2-Dicarboxy-2-hydroxyethyl)-iminodiessigsäure und den Alkalimetallsalzen, insbesondere Na Salzen, davon. Erfindungsgemäß ist es weiterhin bevorzugt, wenn das schweißhemmende kosmetische Mittel zusätzlich mindestens ein Konservierungsmittel enthält. Erfindungsgemäß bevorzugte Konservierungsmittel sind Formaldehydabspalter lodopropinylbutylcarbamate, Parabene, Phenoxyethanol, Ethanol, Benzoesäure und deren Salze, Dibromdicyanobutan, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, . Weitere im Rahmen der vorliegenden Erfindung einsetzbare Konservierungsmittel sind die in Anlage 6 der Kosmetikverordnung aufgeführten Substanzen sowie kosmetische Rohstoffe mit konservierenden Eigenschaften oder Rohstoffe, welche die konservierende Wirkung der vorgenannten Konservierungsmittel unterstützen bzw. verstärken. Die Konservierungsmittel sind vorzugsweise in einer Gesamtmenge von 0,01 bis 10 Gew.-%, vorzugsweise von 0,1 bis 7 Gew.-%, bevorzugt von 0,2 bis 5 Gew.-%, insbesondere von 0,3 bis 2,0 Gew.-%, bezogen auf das Gesamtgewicht des schweißhemmenden kosmetischen Mittels, enthalten.

Im Rahmen der vorliegenden Erfindung ist es bevorzugt, wenn das schweißhemmende kosmetische Mittel als Wasser-in-ÖI-Emulsion vorliegt. Hierbei kann es sich insbesondere um eine versprühbare Wasser-in-ÖI-Emulsion handeln, welche mittels eines Treibmittels versprüht werden kann.

Es kann jedoch erfindungsgemäß gleichermaßen bevorzugt sein, wenn das schweißhemmende kosmetische Mittel als Öl-in-Wasser-Emulsion vorliegt. In diesem Fall wird das erfindungsgemäße kosmetische Mittel bevorzugt als treibmittelfreies Pumpspray oder Quetschspray versprüht oder als Roll-on appliziert.

Gemäß einer weiteren bevorzugten Ausführungsform der vorliegenden Erfindung können die erfindungsgemäßen kosmetischen Mittel nur einen geringen Gehalt an freiem Wasser bzw. kein freies Wasser enthalten. Unter freiem Wasser im Sinne der vorliegenden Erfindung wird Wasser verstanden, welches von Kristallwasser, Hydratationswasser oder ähnlich molekular gebundenem Wasser der eingesetzten Bestandteile verschieden ist. Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels.

Im Rahmen einer weiteren Ausführungsform ist es jedoch erfindungsgemäß ebenfalls bevorzugt, wenn das schweißhemmende kosmetische Mittel als wässrige, wässrig-alkoholische oder wässrigglykolische Lösung vorliegt.

Im Zusammenhang mit dieser Ausführungsform der vorliegenden Erfindung wurde überraschenderweise festgestellt, dass die schweißhemmende Wirkung signifikant gesteigert werden kann, wenn die erfindungsgemäßen schweißhemmenden kosmetischen Mittel freies Wasser in einer Menge von 5 bis 99 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 5 bis 96 Gew.-%, vorzugsweise von 15 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Weiterhin ist es im Zusammenhang mit dieser Ausführungsform bevorzugt, wenn das schweißhemmende kosmetische Mittel Ethanol in einer Gesamtmenge von 1 bis 99 Gew.-%, vorzugsweise von 5 bis 70 Gew.-%, bevorzugt von 7 bis 50 Gew.-%, insbesondere von 10 bis 30 Gew.-%, bezogen auf das Gesamtgewicht des schweißhemmenden kosmetischen Mittels, enthält. Die Applikation des erfindungsgemäßen schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält. Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Zerstäubung des schweißhemmenden kosmetischen Mittels kann hierbei physikalisch, mechanisch oder elektromechanisch, beispielsweise durch Piezzoeffekte oder elektrische Pumpen, erfolgen. Das schweißhemmende kosmetische Mittel kann weiterhin bevorzugt als Stift, Soft Solid, Creme, Gel, Roll-on, loses oder kompaktes Puder konfektioniert sein. Die Formulierung der erfindungsgemäßen schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die erfindungsgemäßen schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten. Weiterhin werden unter mehrphasigen erfindungsgemäßen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können. Die Applikation kann beispielsweise mit einem Rollkugelapplikator oder mittels eines festen Stifts erfolgen.

Es kann im Rahmen der vorliegenden Erfindung ebenfalls bevorzugt sein, wenn das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem erfindungsgemäßen schweißhemmenden kosmetischen Mittel imprägniert oder beaufschlagt und bevorzugt einzeln verpackt. Bevorzugte Substratmaterialien sind ausgewählt aus porösen flächigen Tüchern. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines erfindungsgemäßen schweißhemmenden kosmetischen Mittels auf ein Substrat erhalten werden. Erfindungsgemäß bevorzugt enthält das schweißhemmende kosmetische Mittel mindestens einen weiten Hilfsstoff, ausgewählt aus der Gruppe von (i) Emulgatoren und/oder Tensiden; (ii) Verdickungsmitteln;(iii) Deodorant-Wirkstoffen; (iv) ein- und/oder mehrwertigen Alkoholen und/oder Polyethylenglycolen; (v) hautkühlenden Wirkstoffen; (vi) pH-Stellmitteln; (vii) hautpflegenden Wirkstoffen, wie Moisturizern, hautberuhigenden Stoffen, hautaufhellenden Stoffen, hautglättenden Stoffen; sowie (viii) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear.

Zur Verdickung der erfindungsgemäßen schweißhemmenden kosmetischen Mittel werden bevorzugt Substanzen eingesetzt, welche ausgewählt sind aus Celluloseethern, Xanthan-Gum, Sclerotium Gum, Succinoglucanen, Polygalactomannanen, Pectinen, Agar, Carragheen (Carrageenan), Traganth, Gummi arabicum, Karayagummi, Taragummi, Gellan, Gelatine, Propylenglycolalginat, Alginsäuren und deren Salze, Polyvinylpyrrolidonen, Polyvinylalkoholen, Polyacrylamiden, physikalisch (z. B. durch Vorverkleisterung) und/oder chemisch modifizierten Stärken, Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten. Besonders bevorzugte Verdickungsmittel sind weiterhin ausgewählt aus Carbomeren. Carbomere sind verdickende vernetzte Polymere aus Acrylsäure, Methacrylsäure sowie deren Salzen. Die Vernetzung kann mittels polyfunktioneller Verbindungen wie Polyalkylenether von Polysacchariden oder Polyalkoholen, beispielsweise Sucroseallylether, Pentaerythritolallylether, Propylenallylether, erfolgen. Bevorzugt im Rahmen der vorliegenden Erfindung sind Homopolymere von Acrylsäure oder deren Salzen, welche mit einem Pentaerythritolallylether, einem Sucroseallylether oder einem Propylenallylether vernetzt sind. Ein im Rahmen der vorliegenden Erfindung einsetzbares Verdickungsmittel ist ein Copolymer aus C₁₀₋₃₀-Alkylacrylat, Acrylsäure, Methacrylsäure sowie deren Estern, welches mit einem Sucroseallylether oder einem Pentaerythritolallylether vernetzt ist. Verdickungsmittel auf Basis von Carbomeren sind die unter dem Handelsnamen Carbopol® (BF Goodrich, Ohio, USA) erhältlichen Produkte wie beispielsweise Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, oder Carbopol ultrez 21.

Weiterhin können zur Verdickung der erfindungsgemäßen schweißhemmenden kosmetischen Mittel lipophile Verdickungsmittel eingesetzt werden. Erfindungsgemäß bevorzugte lipophile Verdickungsmittel sind ausgewählt aus hydrophobierten Tonmineralien, Bentoniten, pyrogenen Kieselsäuren sowie deren Derivaten.

Die desodorierende Wirkung der erfindungsgemäßen schweißhemmenden kosmetischen Mittel kann weitergehend gesteigert werden, wenn mindestens ein Deodorant-Wirkstoff mit antibakterieller und/oder bakteriostatischer und/oder enzyminhibierender und/oder geruchsneutralisierender und/oder geruchsabsorbierender Wirkung in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des erfindungsgemäßen schweißhemmenden kosmetischen Mittels, enthalten ist. Sofern Ethanol in den erfindungsgemäßen Mitteln eingesetzt wird, gilt dieses im Rahmen der vorliegenden Erfindung nicht als Deodorant-Wirkstoff, sondern als Bestandteil des Trägers.

Bevorzugte erfindungsgemäße schweißhemmenden kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Als pH-Stellmittel kommen erfindungsgemäß bevorzugt Säuren und/oder Alkalisierungsmittel und/oder Puffer zum Einsatz. Als Säuren werden erfindungsgemäß bevorzugt anorganische Säuren (wie beispielsweise Salzsäure, Schwefelsäure oder Phosphorsäure) oder organische Säuren (wie beispielsweise Zitronensäure, Weinsäure oder Apfelsäure) eingesetzt. Die erfindungsgemäß verwendbaren Alkalisierungsmittel werden bevorzugt ausgewählt aus der Gruppe, welche gebildet wird, aus Ammoniak, basischen Aminosäuren, Alkalihydroxiden, Carbonaten und Hydrogencarbonaten, Alkanolaminen, beispielsweise Amino-2-methyl-1-propanol, Monoethanolamin, Triethanolamin, Diethanolamin und Triisopropanolamin, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Als Puffersysteme eignen sich im Rahmen der vorliegenden Erfindung insbesondere Kohlensäure-Bicarbonat-Puffer, Kohlensäure-Silicat-Puffer, Essigsäure-Acetat-Puffer, Phosphatpuffer, Ammoniakpuffer, Citronensäure- oder Citratpuffer, Puffer auf Basis von Tris(hydroxymethyl)-aminomethan, Puffer auf Basis von 4-(2-Hydroxyethyl)-1-piperazinethanesulfonsäure, Puffer auf Basis von 4-(2-Hydroxyethyl)-piperazin-1-propansulfonsäure, Puffer auf Basis von 2-(N-Morpholino)ethansulfonsäure sowie Barbital-AcetatPuffer. Die Wahl des entsprechenden Puffersystems richtet sich hierbei nach dem gewünschten pH-Wert der erfindungsgemäßen schweißhemmenden kosmetischen Mittel.

Im Rahmen der vorliegenden Erfindung kann es auch vorgesehen sein, das erfindungsgemäße kosmetische Mittel als Zwei-Komponenten-Mittel zu konfektionieren. Die einzelnen Komponenten werden hierzu vorzugsweise in getrennten Containern gelagert und in beliebiger Reihenfolge nacheinander oder gleichzeitig auf die Haut aufgetragen. Eine Auftrennung in Mehrkomponentensysteme ist insbesondere dort bevorzugt, wo Inkompatibilitäten der Inhaltsstoffe zu erwarten oder zu befürchten sind.

Ein weiterer Gegenstand der vorliegenden Erfindung ist daher eine Verpackungseinheit (Kit-of-Parts), umfassend - getrennt voneinander konfektioniert -
a) mindestens einen ersten Container (C1), enthaltend ein kosmetisches Mittel (M1) umfassend mindestens eine schweißhemmende Verbindung, und
b) mindestens einen zweiten Container (C2), enthaltend ein kosmetisches Mittel (M2) umfassend mindestens Salicylsäure und mindestens eine Verbindung der Formel (I), wobei das kosmetische Mittel (M2) keine schweißhemmende Verbindung enthält.

Unter dem Begriff "schweißhemmende Verbindung" sind im Rahmen der vorliegenden Erfindung die zuvor angeführten Halogenide und/oder Hydroxyhalogenide von Aluminium und/oder Zirconium, Verbindungen der Formeln Al(OH)_{y}X und Zr(OH)_{z}X verstanden, wobei X in den vorgenannten Formeln für ein Halogenidion steht, sowie Komplexe aus Halogeniden und/oder Hydroxyhalogeniden des Aluminiums und/oder Zirconiums mit Liganden, wie beispielsweise Aminosäuren, Betainen und Hydroxyalkansäuren, zu verstehen.

Bezüglich des kosmetischen Mittels (M1) in dem Container (C1) und des kosmetischen Mittels (M2) in dem Container (C2) gilt mutatis mutandis das zu den erfindungsgemäßen kosmetischen Mitteln Gesagte.

Schließlich ist ein weiterer Gegenstand der vorliegenden Erfindung ein nicht-therapeutisches kosmetisches Verfahren zur Verhinderung und/oder Reduzierung der Transpiration des Körpers, bei welchem ein erfindungsgemäßes schweißhemmendes kosmetisches Mittel oder der Inhalt einer erfindungsgemäßen Verpackungseinheit auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.

Falls im Rahmen des erfindungsgemäßen Verfahrens die Verpackungseinheit gemäß der Erfindung eingesetzt wird, kann es vorgesehen sein, dass zunächst das kosmetische Mittel (M1) in dem Container (C1) und anschließend das kosmetische Mittel (M2) in dem Container (C2) aufgetragen wird. Es ist jedoch auch möglich, dass zunächst das kosmetische Mittel (M2) in dem Container (C2) und anschließend das kosmetische Mittel (M1) in dem Container (C1) aufgetragen wird. Darüber hinaus können das kosmetische Mittel (M1) in dem Container (C1) und das kosmetische Mittel (M2) in dem Container (C2) auch gleichzeitig aufgetragen werden. Die Zeitspanne zwischen der Anwendung der beiden Mittel (M1) und (M2) beträgt von 0 Sekunden bis 24 Stunden.

Bezüglich des erfindungsgemäßen Verfahrens gilt mutatis mutandis das zu den erfindungsgemäßen schweißhemmenden kosmetischen Mitteln sowie zu der erfindungsgemäßen Verwendung Gesagte.

### Beispiele (ALLE nicht erfindungsgemäss)

**1. Antitranspirant Roll-On - Emulsionen**

| | **1.1** | **1.2** | **1.3** | **1.4.** |
|---|---|---|---|---|
| | Gew-% | Gew-% | Gew-% | Gew-% |
| PPG-15 Stearyl Ether | 0,5 | 0,5 | 0,5 | 0,5 |
| Steareth-2 | 2,5 | 2,5 | 2,5 | 2,5 |
| Steareth-21 | 1,0 | 1,0 | 1,0 | 1,0 |
| Cetearyl Alkohol (Lanette O, BASF) | 0,5 | 0,5 | - | 0,5 |
| Dimethicone (Xiameter PMX-200 Sil Fluid 5CS) | 3% | 3% | - | 3% |
| Salicylsäure | 0,5% | 0,5% | 0,2% | 0,5% |
| Aluminium Sesquichlorohydrate (Reach 301L, 40% in Wasser) | 25,0 | - | - | - |
| Aluminium Chlorohydrat (Locron L von Clariant, 50% in Wasser) | - | 27,0 | 27,0 | - |
| Aluminium Zirkonium Tetrachlorohydrex (AZG 364 von Summit Reheis) | - | - | - | 23,0 |
| EDTA BX Powder (BASF) | 0,1 | 0,1 | 0,1 | 0,1 |
| Parfum | 1,0 | 1,0 | 1,0 | 1,0 |
| Wasser | Ad 100 | Ad 100 | Ad 100 | Ad 100 |

**2. Antitranspirant Roll-On - Alkoholische Lösungen**

| | **2.1** | **2.2** | **2.3** | **2.4.** |
|---|---|---|---|---|
| | Gew-% | Gew-% | Gew-% | Gew-% |
| Ethanol (96%-ig) | 30,0 | 30,0 | 30,0 | 30,0 |
| Hydroxyethylcellulose (Tylose H) | 0,4 | 0,4 | 0,4 | 0,4 |
| Ceteareth-12 (Eumulgin B1, BASF) | 2,0 | 2,0 | 2,0 | 2,0 |
| Ceteareth-30 (Eumulgin B3, BASF) | 2,0 | 2,0 | 2,0 | 2,0 |
| Salicylsäure | 0,5% | 0,5% | 0,2% | 0,5% |
| Aluminium Chlorohydrat (Locron L von Clariant, 50% in Wasser) | 16,0 | 40,0 | 16,0 | - |
| Aluminium Zirkonium Tetrachlorohydrex (AZG 364 von Summit Reheis) | - | - | - | 23,0 |
| EDTA BX Powder (BASF) | 0,1 | 0,1 | 0,1 | 0,1 |
| Parfum | 1,0 | 1,0 | 1,0 | 1,0 |
| Wasser | Ad 100 | Ad 100 | Ad 100 | Ad 100 |

**3. Antitranspirant Aerosol**

| | **3.1** | **3.2** | **3.3** |
|---|---|---|---|
| | Gew-% | Gew-% | Gew-% |
| Cyclopentasiloxane (Xiamater PMX-0245) | 10,7 | 10,7 | 10,7 |
| Dow Corning ES-5227 DM¹ | 5,6 | 5,6 | 5,6 |
| Ispropyl Myristate (BASF) | 6,6 | 6,6 | 6,6 |
| Propylenglycol | 23,4 | 23,4 | 23,4 |
| Phenoxyethanol | 0,5 | 0,5 | 0,5 |
| Aluminum Sesquichlorohydrate (Reach 301L, 40% in Wasser) | 20,0 | 25,0 | 30,0 |
| Aluminium Chlorohydrat (Locron L von Clariant, 50% in Wasser) | | | |
| Salicylsäure | 0,2 | 0,5 | 1,0 |
| EDTA BX Powder (BASF) | 0,05 | 0,1 | 0,15 |
| Parfum | 2,5 | 2,5 | 2,5 |
| Wasser | 29,7 | 24,7 | 18,7 |

| | | | |
|---|---|---|---|
| ¹ 25% PEG/PPG-18/18 Dimethicone / 85% Dimethicone 5cts | | | |

Die Rezepturen 3.1, 3.2 und 3.3 werden im Gewichtsverhältnis von 1 : 4 mit dem Treibmittel Propan/Butan (15/85) in Aerosoldosen abgefüllt.

**4. Klares Gel**

| | **4.1** | **4.2** | **4.3** |
|---|---|---|---|
| Aluminium Chlorohydrat (Locron L von Clariant, 50% in Wasser) | 32,8 | 32,8 | 32,8 |
| Ethanol (96%-ig) | 11,0 | 11,0 | 11,0 |
| Xiameter PMX-2147 Fluid | 7,0 | 7,0 | 7,0 |
| Dow Corning 5225 Formulation Aid | 9,0 | 9,0 | 9,0 |
| Propylenglycol | 6,0 | 6,0 | 6,0 |
| Salicylsäure | 0,2 | 0,5 | 1,0 |
| EDTA BX Powder (BASF) | 0,05 | 0,1 | 0,15 |
| Parfum | 0,5 | 0,5 | 0,5 |
| Wasser | Ad 100 | Ad 100 | Ad 100 |

## Patentansprüche

1. Schweißhemmendes kosmetisches Mittel, enthaltend - jeweils bezogen auf das Gesamtgewicht des schweißhemmenden kosmetischen Mittels -
a) 1 bis 30 Gew.-% mindestens eine schweißhemmende Verbindung,
b) 0,1 bis 10 Gew.-% Salicylsäure
c) mindestens eine Verbindung der Formel (I) 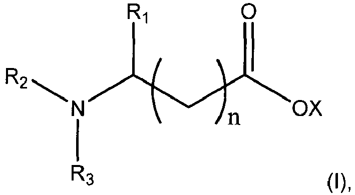 in der
n für 0 oder 1 steht,
X für -H, Na⁺, K⁺ oder NH4⁺ steht,
R₁ ausgewählt ist aus-H, -COOX oder -(CH₂)-COOX steht,
R₂ ausgewählt ist aus -H, C₁₋₁₀-Alkyl oder COOX,
R₃ ausgewählt ist aus -(CH₂)-COOX, -CH(R₁)-COOX, -(CH₂)₂-NH[CH(COOX)(CH₂COOX)], -CH(R₄)-C(R₅)(R₆)-COOX
R₄ ausgewählt ist aus -H, C₁₋₁₀-Alkyl oder COOX,
R₅, R₆ unabhängig voneinander -H, -OH oder C₁₋₁₀-Alkyl sind.

2. Schweißhemmendes kosmetisches Mittel nach Anspruch 1, **dadurch gekennzeichnet, daß** es 2 bis 25 Gew.-%, vorzugsweise 3 bis 24 Gew.-%, bevorzugt 4 bis 23 Gew.-%, weiter bevorzugt 5 bis 20 Gew.-%, noch mehr bevorzugt 6 bis 19 Gew.-% und insbesondere 8 bis 18 Gew.-% Aluminium-Chlorohydrat enthält.

3. Schweißhemmendes kosmetisches Mittel nach einem der Ansprüche 1 oder 2, **dadurch gekennzeichnet, dass** es 2,5 bis 29 Gew.-%, vorzugsweise 5 bis 28 Gew.-%, bevorzugt 7,5 bis 27 Gew.-%, weiter bevorzugt 10 bis 26 Gew.-%, noch mehr bevorzugt 12,5 bis 25 Gew.-% und insbesondere 15bis 23 Gew.-% Aluminium-Zirkonium-Chlorohydrat enthält.

4. Schweißhemmendes kosmetisches Mittel nach einem der Ansprüche 1 bis 3, **dadurch gekennzeichnet, daß** es 0,11 bis 8 Gew.-%, vorzugsweise 0,12 bis 6 Gew.-%, bevorzugt 0,13 bis 4 Gew.-%, weiter bevorzugt 0,14 bis 3 Gew.-%, noch mehr bevorzugt 0,15 bis 2 Gew.-% und insbesondere 0,2 bis 1 Gew.-% Salicylsäure enthält.

5. Schweißhemmendes kosmetisches Mittel nach einem der Ansprüche 1 bis 4, **dadurch gekennzeichnet, daß** es 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (Ib) enthält 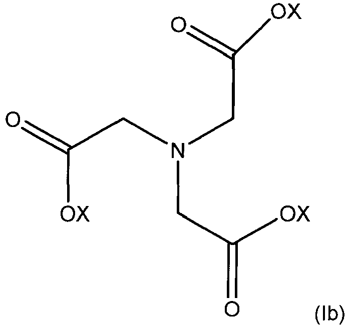 in der X für -H, Na⁺, K⁺ oder NH4⁺ steht.

6. Schweißhemmendes kosmetisches Mittel nach einem der Ansprüche 1 bis 4, **dadurch gekennzeichnet, daß** es 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (Ic) enthält 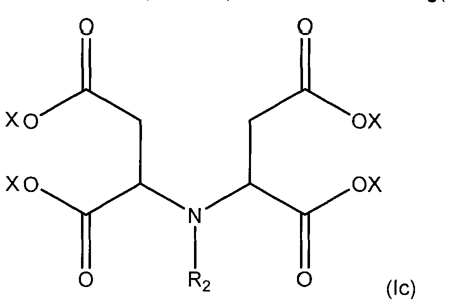 in der
X für -H, Na⁺, K⁺ oder NH4⁺ steht.
R₂ ausgewählt ist aus -H oder -(CH₂)-COOX.

7. Schweißhemmendes kosmetisches Mittel nach einem der Ansprüche 1 bis 4, **dadurch gekennzeichnet, daß** es 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (Id) enthält 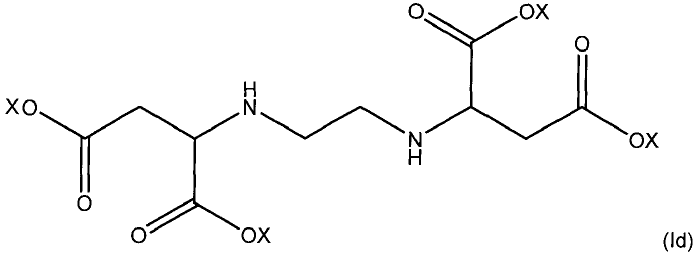 in der
X für -H, Na⁺, K⁺ oder NH4⁺ steht.

8. Schweißhemmendes kosmetisches Mittel nach einem der Ansprüche 1 bis 4, **dadurch gekennzeichnet, daß** es 0,012 bis 10 Gew.-%, vorzugsweise 0,05 bis 5 Gew.-%, bevorzugt 0,1 bis 2,5 Gew.-%, weiter bevorzugt 0,15 bis 1 Gew.-%, noch mehr bevorzugt 0,2 bis 0,75 Gew.-% und insbesondere 0,25 bis 0,5 Gew.-% Verbindung(en) der Formel (le) enthält 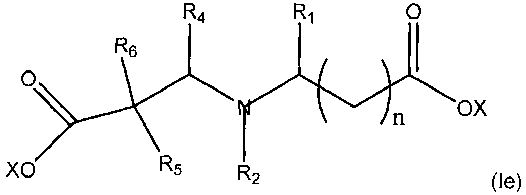 in der
n für 0 oder 1 steht,
X für -H, Na⁺, K⁺ oder NH4⁺ steht,
R₁ für -H oder -(CH₂)-COOX steht
R₂ ausgewählt ist aus -H oder -(CH₂)-COOX,
R₄ ausgewählt ist aus -H, C₁₋₁₀-Alkyl oder COOX,
Rs, R₆ unabhängig voneinander -H, -OH oder C₁₋₁₀-Alkyl sind.

9. Schweißhemmendes kosmetisches Mittel nach Anspruch 8, **dadurch gekennzeichnet, daß** die Verbindung(en) der Formel (Id) ausgewählt ist/sind aus der Gruppe N-(1,2-Dicarboxy-2-hydroxyethyl)-glycin, N-(1,2-Dicarboxy-2-hydroxyethyl)-alanin, N,N-Biscarboxymethyl-β-alanin, N-(1,2-Dicarboxy-2-hydroxyethyl)-sarkosin, N-(1,2-Dicarboxy-2-hydroxyethyl)-iminodiessigsäure und den Alkalimetallsalzen, insbesondere Na Salzen, davon.

10. Nicht-therapeutisches kosmetisches Verfahren zur Verhinderung und/oder Reduzierung der Transpiration des Körpers, bei welchem ein schweißhemmendes kosmetisches Mittel nach einem der Ansprüche 1 bis 9 auf die Haut, insbesondere auf die Haut der Achselhöhlen, aufgetragen wird und für mindestens 1 Stunde, vorzugsweise für mindestens 2 Stunden, bevorzugt für mindestens 4 Stunden, insbesondere für mindestens 6 Stunden, auf der Haut der Achselhöhlen verbleibt.

## Claims

1. An antiperspirant cosmetic agent containing, in each case based on the total weight of the antiperspirant cosmetic agent:
a) 1 to 30 wt.% of an antiperspirant compound,
b) 0.1 to 10 wt.% salicylic acid,
c) at least one compound of formula (I) 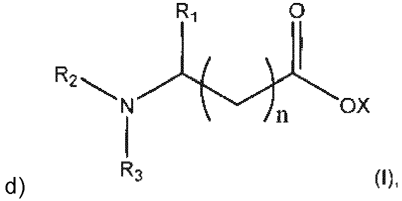 in which
n represents 0 or 1,
X represents -H, Na⁺, K⁺ or NH4⁺,
R₁ is selected from -H, -COOX or -(CH₂)-COOX,
R₂ is selected from -H, C₁₋₁₀-alkyl or COOX,
R₃ is selected from -(CH₂)-COOX, -CH(R₁)-COOX, -(CH₂)₂-NH[CH(COOX)(CH₂COOX)], -CH(R₄)-C(R₅)(R₆)-COOX,
R₄ is selected from -H, C₁₋₁₀-alkyl or COOX,
R₅ and R₆ are, independently of one another,-H, -OH or C₁₋₁₀-alkyl.

2. The antiperspirant cosmetic agent according to claim 1, **characterized in that** it contains 2 to 25 wt.%, preferably 3 to 24 wt.%, preferably 4 to 23 wt.%, more preferably 5 to 20 wt.%, even more preferably 6 to 19 wt.% and in particular 8 to 18 wt.% aluminum chlorohydrate.

3. The antiperspirant cosmetic agent according to one of claims 1 or 2, **characterized in that** it contains 2.5 to 29 wt.%, preferably 5 to 28 wt.%, preferably 7.5 to 27 wt.%, more preferably 10 to 26 wt.%, even more preferably 12.5 to 25 wt.% and in particular 15 to 23 wt.% aluminum-zirconium chlorohydrate.

4. The antiperspirant cosmetic agent according to one of claims 1 to 3, **characterized in that** it contains 0.11 to 8 wt.%, preferably 0.12 to 6 wt.%, preferably 0.13 to 4 wt.%, more preferably 0.14 to 3 wt.%, even more preferably 0.15 to 2 wt.% and in particular 0.2 to 1 wt.% salicylic acid.

5. The antiperspirant cosmetic agent according to one of claims 1 to 4, **characterized in that** it contains 0.012 to 10 wt.%, preferably 0.05 to 5 wt.%, preferably 0.1 to 2.5 wt.%, more preferably 0.15 to 1 wt.%, even more preferably 0.2 to 0.75 wt.% and in particular 0.25 to 0.5 wt.% compound(s) of formula (Ib) 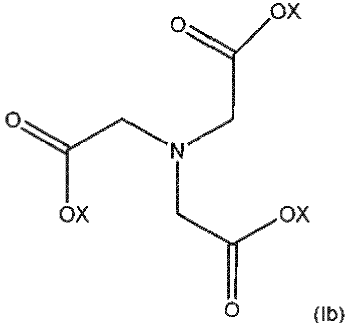 in which
X represents -H, Na⁺, K⁺ or NH4⁺.

6. The antiperspirant cosmetic agent according to one of claims 1 to 4, **characterized in that** it contains 0.012 to 10 wt.%, preferably 0.05 to 5 wt.%, preferably 0.1 to 2.5 wt.%, more preferably 0.15 to 1 wt.%, even more preferably 0.2 to 0.75 wt.% and in particular 0.25 to 0.5 wt.% compound(s) of formula (Ib) 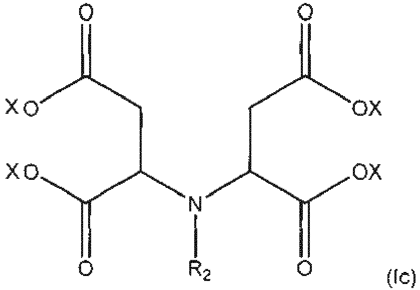 in which
X represents -H, Na⁺, K⁺ or NH4⁺.
R₂ is selected from -H or -(CH₂)-COOX.

7. The antiperspirant cosmetic agent according to one of claims 1 to 4, **characterized in that** it contains 0.012 to 10 wt.%, preferably 0.05 to 5 wt.%, preferably 0.1 to 2.5 wt.%, more preferably 0.15 to 1 wt.%, even more preferably 0.2 to 0.75 wt.% and in particular 0.25 to 0.5 wt.% compound(s) of formula (Id) 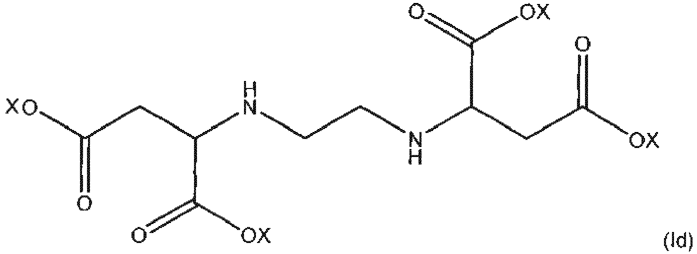 in which
X represents -H, Na⁺, K⁺ or NH4⁺.

8. The antiperspirant cosmetic agent according to one of claims 1 to 4, **characterized in that** it contains 0.012 to 10 wt.%, preferably 0.05 to 5 wt.%, preferably 0.1 to 2.5 wt.%, more preferably 0.15 to 1 wt.%, even more preferably 0.2 to 0.75 wt.% and in particular 0.25 to 0.5 wt.% compound(s) of formula (le) 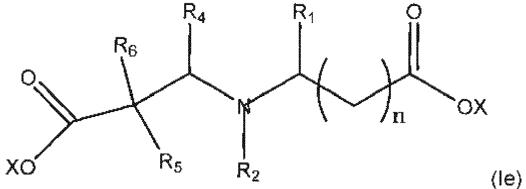 in which
n represents 0 or 1,
X represents -H, Na⁺, K⁺ or NH4⁺,
R₁ represents -H or -CH₂-COOX,
R₂ is selected from -H or -(CH₂)-COOX,
R₄ is selected from -H, C₁₋₁₀-alkyl or COOX,
R₅ and R₆ are, independently of one another,-H, -OH or C₁₋₁₀-alkyl.

9. The antiperspirant cosmetic agent according to claim 8, **characterized in that** the compound(s) of formula (Id) is/are selected from the group N-(1,2-dicarboxy-2-hydroxyethyl)glycine, N-(1,2-dicarboxy-2-hydroxyethyl)alanine, N-biscarboxymethyl-β-alanine, N-(1,2-dicarboxy-2-hydroxyethyl)sarcosine, N-(1,2-dicarboxy-2-hydroxyethyl)iminodiacetic acid and the alkali metal salts, in particular Na salts, thereof.

10. A non-therapeutic cosmetic method for preventing and/or reducing body perspiration, in which an antiperspirant cosmetic agent according to one of claims 1 to 9 is applied to the skin, in particular to the skin of the armpits, and remains on the skin of the armpits for at least 1 hour, preferably for at least 2 hours, more preferably for at least 4 hours, in particular for at least 6 hours.

## Revendications

1. Agent cosmétique antisudoral contenant, dans chaque cas par rapport au poids total de l'agent cosmétique antisudoral,
a) 1 à 30 % en poids d'au moins un composé antisudoral,
b) 0,1 à 10 % en poids d'acide salicylique,
c) au moins un composé de formule (I) 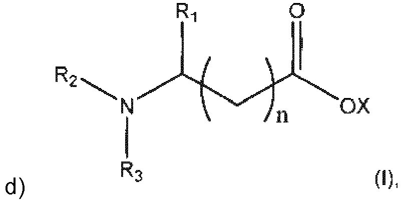 dans laquelle
n représente 0 ou 1,
X représente -H, Na⁺, K⁺ ou NH4⁺,
R₁ est choisi parmi -H, -COOX ou -(CH₂)-COOX,
R₂ est choisi parmi -H, un alkyle en C₁₋₁₀ ou COOX,
R₃ est choisi parmi -(CH₂)-COOX, -CH(R₁)-COOX, -(CH₂)₂-NH[CH(COOX)(CH₂COOX)], -CH(R₄)-C(R₅)(R₆)-COOX,
R₄ est choisi parmi -H, un alkyle C₁₋₁₀ ou COOX,
R₅ et R₆ représentent indépendamment l'un de l'autre -H, -OH ou un alkyle en C₁₋₁₀.

2. Agent cosmétique antisudoral selon la revendication 1, **caractérisé en ce qu'**il contient 2 à 25 % en poids, de préférence 3 à 24 % en poids, de préférence 4 à 23 % en poids, de manière davantage préférée 5 à 20 % en poids, de manière encore plus préférée 6 à 19 % en poids et en particulier 8 à 18 % en poids de chlorhydrate d'aluminium.

3. Agent cosmétique antisudoral selon l'une des revendications 1 ou 2, **caractérisé en ce qu'**il contient 2,5 à 29 % en poids, de préférence 5 à 28 % en poids, de préférence 7,5 à 27 % en poids, de manière davantage préférée 10 à 26 % en poids, de manière encore plus préférée 12,5 à 25 % en poids et en particulier 15 à 23 % en poids de chlorhydrate de zirconium-aluminium.

4. Agent cosmétique antisudoral selon l'une des revendications 1 à 3, **caractérisé en ce qu'**il contient 0,11 à 8 % en poids, de préférence 0,12 à 6 % en poids, de préférence 0,13 à 4 % en poids, de manière davantage préférée 0,14 à 3 % en poids, de manière encore plus préférée 0,15 à 2 % en poids et en particulier 0,2 à 1 % en poids d'acide salicylique.

5. Agent cosmétique antisudoral selon l'une des revendications 1 à 4, **caractérisé en ce qu'**il contient 0,012 à 10 % en poids, de préférence 0,05 à 5 % en poids, de préférence 0,1 à 2,5 % en poids, de manière davantage préférée 0,15 à 1 % en poids, de manière encore plus préférée 0,2 à 0,75 % en poids et en particulier 0,25 à 0,5 % en poids de composé(s) de formule (Ib) 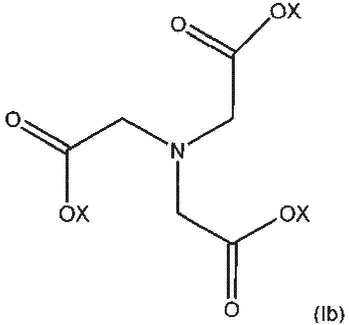 dans laquelle
X représente -H, Na⁺, K⁺ ou NH4⁺.

6. Agent cosmétique antisudoral selon l'une des revendications 1 à 4, **caractérisé en ce qu'**il contient 0,012 à 10 % en poids, de préférence 0,05 à 5 % en poids, de préférence 0,1 à 2,5 % en poids, de manière davantage préférée 0,15 à 1 % en poids, de manière encore plus préférée 0,2 à 0,75 % en poids et en particulier 0,25 à 0,5 % en poids de composé(s) de formule (Ic) 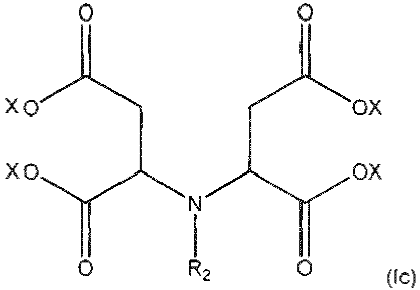 dans laquelle
X représente -H, Na⁺, K⁺ ou NH4⁺.
R₂ est choisi parmi -H ou -(CH₂)-COOX.

7. Agent cosmétique antisudoral selon l'une des revendications 1 à 4, **caractérisé en ce qu'**il contient 0,012 à 10 % en poids, de préférence 0,05 à 5 % en poids, de préférence 0,1 à 2,5 % en poids, de manière davantage préférée 0,15 à 1 % en poids, de manière encore plus préférée 0,2 à 0,75 % en poids et en particulier 0,25 à 0,5 % en poids de composé(s) de formule (Id) 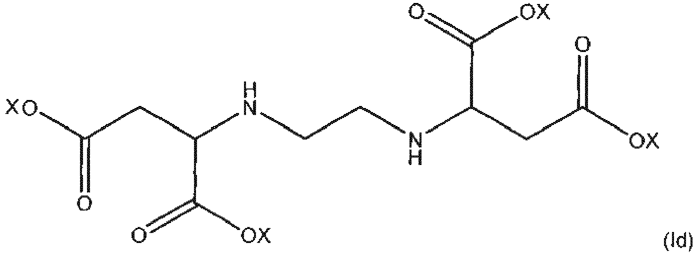 dans laquelle
X représente -H, Na⁺, K⁺ ou NH4⁺.

8. Agent cosmétique antisudoral selon l'une des revendications 1 à 4, **caractérisé en ce qu'**il contient 0,012 à 10 % en poids, de préférence 0,05 à 5 % en poids, de préférence 0,1 à 2,5 % en poids, de manière davantage préférée 0,15 à 1 % en poids, de manière encore plus préférée 0,2 à 0,75 % en poids et en particulier 0,25 à 0,5 % en poids de composé(s) de formule (le) 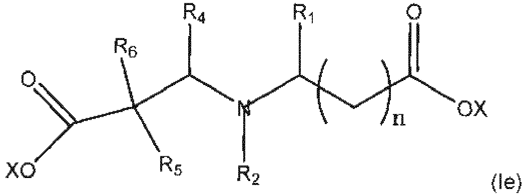 dans laquelle
n représente 0 ou 1,
X représente -H, Na⁺, K⁺ ou NH4⁺,
R₁ représente -H ou -(CH₂)-COOX,
R₂ est choisi parmi -H ou -(CH₂)-COOX,
R₄ est choisi parmi -H, un alkyle en C₁₋₁₀ ou COOX,
R₅ et R₆ représentent indépendamment l'un de l'autre -H, -OH ou un alkyle en C₁₋₁₀.

9. Agent cosmétique antisudoral selon la revendication 8, **caractérisé en ce que** le(s) composé(s) de formule (Id) est/sont choisi(s) dans le groupe constitué par la N-(1,2-dicarboxy-2-hydroxyéthyl)-glycine, la N-(1,2-dicarboxy-2-hydroxyéthyl)-alanine, la N,N-biscarboxyméthyl-β-alanine, la N-(1,2-dicarboxy-2-hydroxyéthyl)-sarcosine, l'acide N-(1,2-dicarboxy-2-hydroxyéthyl)-iminodiacétique et les sels de métaux alcalins, notamment des sels de sodium dérivés de ceux-ci.

10. Procédé cosmétique non thérapeutique destiné à prévenir et/ou réduire la transpiration du corps, dans lequel un agent cosmétique antisudoral selon l'une des revendications 1 à 9 est appliqué sur la peau, en particulier sur la peau des aisselles, et reste sur la peau pendant au moins 1 heure, de préférence pendant au moins 2 heures, de manière davantage préférée pendant au moins 4 heures, en particulier pendant au moins 6 heures sur la peau des aisselles.
